**Europäisches Patentamt**

**European Patent Office**

**Office européen des brevets**

(11) Veröffentlichungsnummer: **0 439 180 B1**

(12) **EUROPÄISCHE PATENTSCHRIFT**

(45) Veröffentlichungstag der Patentschrift: **19.04.95**

(21) Anmeldenummer: **91100927.2**

(22) Anmeldetag: **25.01.91**

(51) Int. Cl.⁶: **A61L 15/44**, A61K 9/70,
A61K 31/135

(54) **Transdermales therapeutisches System mit dem Wirkstoff Tulobuterol.**

(30) Priorität: **26.01.90 DE 4002281**

(43) Veröffentlichungstag der Anmeldung:
**31.07.91 Patentblatt 91/31**

(45) Bekanntmachung des Hinweises auf die
Patenterteilung:
**19.04.95 Patentblatt 95/16**

(84) Benannte Vertragsstaaten:
**AT BE CH DE DK ES FR GB GR IT LI LU NL SE**

(56) Entgegenhaltungen:
**EP-A- 0 156 080**

(73) Patentinhaber: **LTS LOHMANN THERAPIE-SY-
STEME GmbH & CO.KG
Postfach 23 43
D-56513 Neuwied (DE)**

(72) Erfinder: **Hoffmann, Hans-Rainer, Dr.
Burghofstrasse 123
W-5450 Neuwied 22 (DE)**
Erfinder: **Horstmann, Michael, Dr.
Deichstrasse 9
W-5450 Neuwied 1 (DE)**

(74) Vertreter: **Flaccus, Rolf-Dieter, Dr.
Patentanwalt
Sperlingsweg 32
D-50389 Wesseling (DE)**

**Beschreibung**

Die Erfindung betrifft ein wirkstoffhaltiges Hautpflaster zur Behandlung von Asthma bronchiale mit dem Wirkstoff Tulobuterol sowie ein Verfahren zur Herstellung dieses Hautpflasters. Die erfindungsgemäßen Ausführungsformen, welche aus einer im wesentlichen wirkstoffundurchlässigen Rückschicht und einer selbsthaftklebenden wirkstoffhaltigen Matrixschicht bestehen, weisen eine Matrix auf, die wenigstens ein Styrol-1,3-Dien-Styrol-Blockcopolymer enthält. Als besonders bevorzugtes Herstellungsverfahren wird das Aufbringen der wirkstoffhaltigen Klebeschicht mittels eines Heißschmelzverfahrens beschrieben.

Transdermale therapeutische Systeme (TTS) sind auf der Haut aufzubringende selbstklebende galenische Zubereitungen mit festgelegter Applikationsfläche, die einen Arzneistoff nach Zeit und Menge kontrolliert an den menschlichen oder tierischen Körper abgeben. Derartige Systeme, die beispielsweise von Y.W. Chien, Drug Dev. Ind. Pharm. 13, 589-651 (1987), beschrieben sind, haben sich seit Jahren in der Therapie bewährt.

Übliche Bauformen von transdermalen Systemen, die bereits Eingang in die Praxis gefunden haben, sind:

a) Aufbau aus undurchlässigem Träger und einer zweiten, gleichzeitig als Arzneistoffreservoir, Haftkleber und Steuereinheit dienenden Schicht,

b) Aufbau aus Träger, Arzneistoffreservoir, Kontrolleinheit und Klebschicht in räumlicher Trennung,

c) Aufbau aus Träger und mehrschichtig angeordneter arzneistoffhaltiger Matrix, wobei die Wirkstoffkonzentration von Schicht zu Schicht zur Haut hin geringer wird,

d) Aufbau aus Träger und Matrix, wobei die Freisetzung durch in der Matrix dispergierte arzneistoffhaltige Mikrokapseln kontrolliert wird.

Der therapeutische Fortschritt dieser Systeme gegenüber herkömmlichen Applikationsformen besteht darin, daß der Wirkstoff dem Körper nicht stoßweise zugeführt wird, wie beispielsweise bei der Einnahme von Tabletten, sondern kontinuierlich.

Dadurch wird einerseits die Wirkungsdauer eines Arzneistoffes verlängert, zum anderen werden Nebenwirkungen durch Vermeidung unnötiger Blutspiegelspitzen weitgehend verhindert.

Besonders beim Asthma bronchiale als chronischer Erkrankung ist das Erzielen eines medikamentösen Dauerschutzes besonders vorteilhaft.

Zur Asthmatherapie geeignete Wirkstoffe sind ß-Adrenergica (Terbutalin, Salbutamol), Bronchospasmolytica (Theophyllin, Etofyllin), Mastzellstabilisatoren (Cromoglicinsäure, Ketotifen), Parasympatholytica (Ipratropiumbromid) und Corticosteroide (Betametason, Beclometason).

Während sich viele dieser Stoffe bei der Therapie des akuten Asthmaanfalls von Form von Dosieraerosolen gut bewährt haben, ist die Dauertherapie der Erkrankung beispielsweise mit oral angewendeten ß-Adrenergica noch unbefriedigend. Diese Substanzen rufen vor allem bei höheren als therapeutisch notwendigen Blutspiegeln Pulserhöhung und Blutdrucksteigerung hervor. Die Anwendung von transdermalen therapeutischen Systemen wäre gerade für den genannten Indikationsbereich besonders vorteilhaft, zumal es dem Schutzbedürfnis des Asthmatikers entgegenkommt, eine wirksame und erkennbare medikamentöse Dauerprophylaxe mit sich zu führen.

Leider sind jedoch nur wenige Arzneistoffe zum Einsatz in transdermalen therapeutischen Systemen geeignet. Hierzu gibt es eine Reihe von Gründen, unter denen mangelhafte chemisch-strukturelle Eignung, zu hohe therapeutische Tagesdosis, chemische Instabilität nur beispielhaft genannt sind.

So befindet sich unter anderem aus diesem Grunde bis zum jetzigen Zeitpunkt kein Asthma-TTS auf dem Markt.

Aus der DE-OS 37 32 642 ist die transdermale Verabreichung von Terbutalin, aus der EP-A 306 926 von Salbutamol und aus der EP-A 227 836 von Clenbuterol bekannt. Bei diesen Wirkstoffen handelt es sich um $\beta$-Adrenergica, die mit dem Wirkstoff Tulobuterol einige chemische Strukturelemente gemeinsam haben, sich jedoch pharmakokinetisch und galenisch sowie in der erforderlichen therapeutischen Tagesdosis von letzterem wesentlich unterscheiden.

Clenbuterol beispielsweise besitzt eine viel zu lange inhärente Wirkungsdauer (biologische Halbwertzeit ca. 35 Stunden), die bereits bei oraler Gabe Kumulationsrisiken birgt und es für die transdermale Gabe ungeeignet macht.

In der japanischen Offenlegungsschrift 63-10716 ist ein äußerliches Arzneimittel mit einem $\beta$-Stimulans, z.B. Clenbuterol, Salbutamol, Procaterol und Tulobuterol als aktivem Bestandteil beschrieben. Dabei wird Tulobuterol u.a. in einem Acrylat/Methacrylat-Copolymer gelöst, das Gemisch auf einen Baumwollstoff aufgetragen und zur Filmbildung getrocknet, um so einen Pastenaufkleber zu erhalten.

Aus der EP-A 0 374 980 ist eine perkutane Zubereitung von Tulobuterol bekannt, bei der die Wirksubstanz in einer auf einem Träger befindlichen Matrix aus Polyisobutylen enthalten ist. Die Verwendung von Polyisobutylen als Träger des Wirkstoffs soll sich dabei günstig auf die Freisetzungsrate und die Stabilität des Tulobuterols ohne Verwendung anderer Zusätze bekannter Art auswirken.

Gegenüber diesem Stand der Technik liegt der vorliegenden Erfindung die Aufgabe zugrunde, ein zur Asthma-Therapie geeignetes transdermales therapeutisches System mit Tulobuterol als Wirkstoff bereitzustellen, welches eine vereinfachte von Wirkstoffverlusten geschützte Herstellung und Handhabung, eine sichere Dosierung des Wirkstoffes bei optimaler Freisetzungsrate und Verträglichkeit ermöglicht.

Diese Aufgabe wird erfindungsgemäß durch ein transdermales therapeutisches System mit Tulobuterol (2-tert. Butylamino-1-(2'-Chlorphenyl)-Ethanol) oder einem seiner pharmazeutisch verträglichen Salze als Wirkstoff, enthaltend eine im wesentlichen wirkstoffundurchlässige Rückschicht und wenigstens eine den Wirkstoff enthaltende Matrixschicht, wobei die Matrixschicht wenigstens ein Styrol-1,3-Dien-Styrol-Blockcopolymer enthält, gelöst.

Überraschenderweise verfügt der $\beta$-adrenerge Wirkstoff Tulobuterol über eine bisher nicht erkannte Kombination von Eigenschaften, die ihn für die Verwendung in einem TTS geradezu prädestinieren. Hierzu gehören die günstige Kombination von guter Löslichkeit in organischen Lösungsmitteln mit einer noch erhaltenen Wasserlöslichkeit, die bemerkenswerte chemische Stabilität, die selbst bei Temperaturen über 160°C zu keiner kalorimetrisch nachweisbaren Zersetzung führte, und die hohe Wirksamkeit des Stoffes (ca. 3-5 mg pro Tag).

Das erfindungsgemäße transdermale therapeutische System weist bevorzugt eine im wesentlichen wirkstoffundurchlässige Rückschicht sowie wenigstens eine den Wirkstoff enthaltende Matrixschicht auf, die wenigstens ein Styrol-1,3-Dien-Styrol-Blockcopolymer enthält. Das System kann zusätzlich zu der oder den wirkstoffhaltigen Schichten hautseitig auch eine oder mehrere Schichten aufweisen, die im wesentlichen wirkstofffrei sind. Weiterhin können die Schichten des Systems unterschiedlich dick sein und sich im galenischen Aufbau, insbesondere in der Hilfsstoffzusammensetzung, unterscheiden. Der Wirkstoff kann in der Matrixschicht vorzugsweise durch Auflösen homogen verteilt sein, er kann aber ebenso als Feststoff in der Matrix in feinverteilter Suspension vorliegen, wobei Korngrößen des Wirkstoffs zwischen 1 und 100 $\mu$m bevorzugt werden.

Für den Einsatz des Wirkstoffs in TTS mit einer Matrix mit wenigstens einem Styrol-1,3-Dien-Styrol-Blockcopolymer eignen sich dem Fachmann bekannte Hilfsstoffe wie Polymere, klebrigmachende Harze, Antioxidantien, Weichmacher, Füllstoffe, Lösungsvermittler, Aufschmelzhilfsmittel, Emulsionsförderer und andere Stoffe.

Die für die strukturelle mechanische Festigkeit (Kohäsion) wichtigen Polymere sind Styrol-1,3-Dien-Styrol-Blockcopolymere, insbesondere Styrol-Butadien-Styrol- und Styrol-Isopren-Styrol-Blockcopolymere.

Die Beimischung von Copolymeren von Estern und Amiden der Acryl- und Methacrylsäure, Polyvinylestern von Fettsäuren, Polyvinylethyl- oder -isobutyl-ethern, 1,2-Propandiol-Adipinsäure-Ester, natürlichen oder synthetische Kautschuken, Polyolefinen, Isobutylen-Isopren-Mischpolymerisaten, Polyethylen, Cellulosederivaten wie Ethylcellulose oder Celluloseacetatphthalat zu den genannten Blockcopolymeren kann zur Abstimmung der mechanischen Eigenschaften, Klebkraft und Kohäsion von Vorteil sein.

Als klebrigmachende Harze sind beispielsweise Benzoeharz, Dammarharz, Kopal, Montansäureester, Sandarakharz, Schellack, aliphatische Kohlenwasserstoffharze, Ester von (hydriertem) Kolophonium bzw. (hydriertem) Abietylalkohol, Derivate des Beta-Pinen, Polyolefinharzen, Cumaron-Inden-Harze geeignet.

Antioxidantien dienen in der Regel zum Schutz vor dem Einfluß des Luftsauerstoffs - es sind hier beispielhaft genannt:

Butylhydroxianisol, Butylhydroxitoluol, delta-Tocopherol, Gamma-Tocopherol(-acetat), Octylgallat, L-Ascorbinsäure, Ascorbylpalmitat.

Füllstoffe wie z.B. Titandioxid, Kreide, Bentonit, Calciumphosphat, Kaolin, Lactose, kolloidale Kieselsäure, Talkum, Magnesiumcarbonat können ebenso enthalten sein wie in Wasser quellbare Stoffe wie Xanthan, Pektin, Stärke und ihre Derivate, Cellulose und ihre Derivate Carrageen, Dextrin, Traganth, Polyvinylpyrrolidon, Gelatine, Gummi arabicum, Johannisbrotkernmehl und weitere Stoffe sowie Mischungen solcher Materialien.

Beispielhafte Lösungsvermittler und Weichmacher sind Fettsäuren, Triglyceride, Paraffine, Ethyloleat und andere Fettsäureester linearer ein- oder mehrwertiger Alkohole, Octanol und andere mittelkettige Alkohole, Phthalsäureester, Mineralöl, Glycerin, Propylenglykol, Mono- und Diglyceride von Speisefettsäuren, Natriumlaurylsulfat, Polyoxyethylenalkylether, Polyoxyethylen, Lecithin oder Polyoxyethylensorbitanester.

Das erfindungsgemäße TTS wird nach einem ebenfalls zur Erfindung gehörenden Verfahren dadurch hergestellt, daß Kleber, Wirkstoff und sonstige Hilfsstoffe gemeinsam in einem geeigneten Lösemittel gelöst und zum Beispiel durch ein Streichverfahren auf dem Träger aufgebracht werden und diesen anschließend durch Trocknung vom Lösemittel zu befreien und auf eine halbfeste, klebrige Konsistenz zu bringen. Ebenso ist es möglich, die genannte lösemittelhaltige Masse auf eine kleberabweisende (dehäsiv ausgerüstete) Folie zu streichen, in entsprechender Weise zu trocknen und anschließend durch Kaschieren auf den endgültigen Träger zu übertragen.

Dabei kann es auch vorteilhaft sein, mehrere wirkstoffhaltige oder wirkstofffreie Schichten aufeinander zu kaschieren, etwa um außergewöhnlich hohe Flächenkonzentrationen von Wirkstoff in einem TTS zu erzeugen. Im allgemeinen reicht eine Beladung mit 8 bis 30 mg auf einer Fläche von 10 bis 35 cm$^2$ jedoch völlig aus.

Darüber hinaus ist Tulobuterol ein derart robuster Wirkstoff, daß sich auch eine ganze Reihe anderer, spezieller Verfahren des Aufdruckens auf Folie oder Vlies, des Aufsprühens und weiterer Dosierungsmethoden zur Fertigung von TTS eignen.

Besonders soll hier jedoch das sogenannte Heißschmelzverfahren erwähnt werden, bei welchem ohne Verwendung von Lösemitteln Tulobuterol und die Hilfsstoffe miteinander verschmolzen werden und diese gegebenenfalls durch Kneten homogenisierte Masse in der Hitze auf den Träger oder eine dehäsiv ausgerüstete Folie aufgebracht wird. Geeignet zur Herstellung sind zum Beispiel in der Kunststoffverarbeitung bekannte heizbare Extruder mit schlitzförmiger Austrittsdüse. Das Verfahren bietet den Vorteil der Lösemittelersparnis und der Vermeidung hoher Energiekosten, die beim Trocknen lösungsmittelhaltiger Filme anfallen.

Für den erfindungsgemäßen Zweck ist es sowohl möglich, den Wirkstoff vollständig gelöst in die Kleber-Matrix bringen, als auch, einen Teil der Substanz in feinverteilter Suspension in der Grundmasse zu verteilen.

Die Erfindung wird durch die nachfolgenden Beispiele erläutert, die jedoch keine Beschränkung darstellen:

Beispiel 1:

157,3 g Polyisobutylen
relative mittlere Molekülmasse
ca. 1.270.000
(z.B. Oppanol ® B 100)
Lösung (21,3 % g/g) in Benzin
33,7 g Polyisobutylen
relative mittlere Molekülmasse
ca. 40.000
(z.B. Oppanol ® B 10)
16,5 g Polyisobutylen
relative mittlere Molekülmasse
ca. 800
(z.B. Oppanol ® B 3)
16,5 g thermoplastisches Kohlenwasserstoffharz
(z.B. Escorez ® 5300)
werden in
110,7 g n-Hexan
unter Rühren gelöst.
Zu 60,0 g dieser Lösung werden in einem zylindrischen Glasbehälter
0,96 g Tulobuterol-Base
gegeben und die Lösung mit einem Magnetrührstab bis zur vollständigen Auflösung gerührt.

Die Lösung wird auf siliconisierter Polyesterfolie (100 μm dick) in einer Schichtdicke von 300 μm ausgestrichen. Die Trocknung wurde in fünf Stufen vorgenommen, aus jeder Stufe wurde in einem Stanzling der Tulobuterolgehalt mittels Hochdruckflüssigkeitschromatographie (Silicagel, UV-Detektion bei 210 nm) ermittelt:

| Bedingungen: | Gehalt ($mg/cm^2$) |
|---|---|
| Raumtemperatur, 10 Minuten | 0,34 |
| zusätzlich 60 °C, 20 Minuten | 0,29 |
| zusätzlich 80 °C, 10 Minuten | 0,17 |
| zusätzlich 80 °C, 20 Minuten | 0,06 |
| zusätzlich 80 °C, 30 Minuten | 0,01 |

Die für die nahezu vollständige Entfernung des Lösemittels erforderlichen Trocknungsbedingungen ziehen also unweigerlich erhebliche Verdampfungsverluste an Wirkstoff nach sich, die hinsichtlich Dosierungsgenauigkeit und ferner auch Arbeits-und Umweltschutz höchst unerwünscht sind.

Beispiel 2:

0,450 g Tulobuterol-Base
4,47 g festes Harz, Copolymer von Diolefinen und Olefinen
(z.B. Escorez ® 4401)
15,53 g Polystyrol-Polyisopren-Polystyrol-Blockcopolymer
(z.B. Cariflex ® TR 1107)
- 19,2 % ige Lösung in Benzinwerden bis zur vollständigen Auflösung von Wirkstoff und Harz gerührt. Die Lösung wird auf silikonisierte Polyesterfolie (100 Mikrometer) in einer Schichtdicke von 300 Mikrometern ausgestrichen und nach zehnminütigem Ablüften bei Raumtemperatur zwanzig Minuten bei 50 °C nachgetrocknet.
Durch Auswaage errechnet sich ein Flächengewicht der aufgetragenen trockenen Klebmasse von 60 $g/m^2$ entsprechend einem Wirkstoffgehalt von 0,87 $mg/2,54\ cm^2$ bzw. 5,5 $mg/16\ cm^2$. Mit einer Kaschiervorrichtung wird der Träger (15 Mikrometer starke klare Polyesterfolie) aufgebracht.

Auch aus diesem Laminat lassen sich transdermale therapeutische Systeme beliebiger Größe stanzen, die nach Ablösen der silikonisierten Polyesterfolie ein über mindestens 24 h ausreichendes Klebverhalten auf der menschlichen Haut aufweisen.

Beispiel 2a:

4,51 g festes Harz, Copolymer von Diolefinen und Olefinen
(z.B. Escorez ® 4401)
2,99 g Styrol-Isopren-Styrol Blockcopolymer
(z.B. Cariflex ® TR 1107)
werden gemischt und in einem zylindrischen Metallgefäß (Innendurchmesser 36 mm) eine Stunde lang auf einer Temperatur von 140 °C gehalten. Mit einem zylindrischen Schneckenrührer (Außendurchmesser 33 mm) wird die Masse mit 100 Umdrehungen pro Minute gerührt und dabei innerhalb von 30 Minuten auf 90°C abgekühlt. (Diese Masse besitzt eine Viskosität von ca. 2000 dPas, gemessen bei 140 °C; Haake Viskotester VT-02). Nach einer Stunde schnellen Rührens bei 250 Umdrehungen pro Minute werden
0,44 g Tulobuterol-Base
zugegeben. Die Masse wird anschließend 20 Minuten lang bei 110 °C und 250 Umdrehungen pro Minute weitergerührt und es entsteht eine klare, streichfähige Polymermasse (Viskosität ca. 900 dPas bei 120°C). Etwa 5 g dieser Masse werden zwischen zwei siliconisierte Polyesterfolien gebracht und auf 110 °C vorgewärmt (die Masse bleibt jedoch auch bei 80 - 90 °C noch verarbeitbar). Das Sandwich wird durch Ziehen zwischen einer auf 100 °C vorgeheizten Stahlplatte und einer schräg angeschliffenen Stahlkante bei einer Spaltbreite von 400 µm zu einem gleichmäßigen Laminat umgeformt. Durch Auswaage errechnet sich ein Flächengewicht der reinen Klebmasse von 242 $g/m^2$ entsprechend einem Wirkstoffgehalt von 3,4 $mg/2,54\ cm^2$ bzw. 21,4 $mg/16\ cm^2$. Eine der beiden siliconisierten Polyesterfolien wird nun abgezogen und der endgültige Träger (15 µm starke klare Polyesterfolie) durch Kaschieren aufgebracht.

Das hier beschriebene Verfahren schließt Verdampfungsverluste an Wirkstoff von vornherein praktisch aus, da die Klebermasse niemals in dünner Schicht offen zutage liegt. Abgesehen hiervon und von der höheren Schichtdicke entsprechen die Eigenschaften dieser Rezeptur dem Beispiel 2, insbesondere auch hinsichtlich Klebkraft und Kohäsion.

Beispiel 3: Wirkstofffreisetzung:

Von den transdermalen therapeutischen Systemen wurden 16 cm$^2$ große Stanzlinge hergestellt und die Wirkstoffliberation auf nachfolgend beschriebene Weise bestimmt: (Beispiel 1: Stand der Technik; Beispiel 2, 2a gemäß Erfindung; Beispiel 6 Wirkstoff Salbutamol Stand der Technik)
Das TTS wird in einem dicht verschlossenen zylindrischen Glasgefäß in 100 ml physiologische Kochsalzlösung gebracht und bei 37°C unter leichter Agitation (Schüttelwasserbad) inkubiert. Nach 2,4 und 8 Stunden wird das Medium ausgetauscht, die zu diesen Zeitpunkten und nach 24 Stunden anfallenden wäßrigen Lösungen werden auf den Gehalt an Tulobuterol bzw. Salbutamol untersucht. Hierzu dient eine spektralphotometrische Vergleichsmessung der Probenlösungen gegen einen in gleicher Weise und in gleichem Medium hergestellten Wirkstoffstandard bei einer Wellenlänge von 210 nm. Zum Nullpunktabgleich wird physiologische Kochsalzlösung verwendet. Nach Summation der gefundenen Menge wurden folgende Werte erhalten:

| TTS gemäß | nach | mg/16 cm$^2$ freigesetzt | | | |
|---|---|---|---|---|---|
| | | 2h | 4h | 8h | 24h |
| Beispiel 1 | | 2,08 | 2,96 | 4,01 | 4,97 |
| Beispiel 2 | | 2,12 | 3,11 | 4,17 | 4,79 |
| Beispiel 2a | | 2,19 | 3,11 | 4,51 | 8,04 |
| Beispiel 6 (Salbutamol) | | 0,59 | 0,64 | 0,69 | 0,74 |

Beispiel 4: Wirkstoffpermeation durch Tierhaut in vitro:

Von den TTS nach den in Beispiel 3 aufgeführten Proben werden 2,54 cm$^2$ große kreisförmige Stanzlinge hergestellt und die Wirkstoffpermeation durch die isolierte haarlose Mäusehaut in vitro auf folgende Weise bestimmt:
Das TTS wird mittig auf die Außenseite eines Stückes Mäusehaut geklebt und in eine Permeationszelle eingespannt, deren prinzipieller Aufbau z.B. bei Kondo et al., J. Pharmacobio.-Dyn. 10, 662-668 (1987) beschrieben ist. Die verwendete Glasapparatur enthält als Akzeptormedium ca. 20 ml physiologische Kochsalzlösung und wird über einen Temperiermantel auf 37°C gehalten. Nach 8 Stunden wird das Medium ausgetauscht, die zu diesem Zeitpunkt und nach 24 Stunden anfallenden wäßrigen Lösungen werden auf den Gehalt an Tulobuterol bzw. Salbutamol mittels Hochdruckflüssigkeitschromatographie untersucht. Hierzu dient eine Reverse-Phase-Silicagel-Säule mit angeschlossenem UV-Detektor bei einer Wellenlänge von 215 nm. Die Quantifizierung erfolgt über vergleichende Auswertung der Peakflächen gegen einen entsprechend hergestellten Wirkstoffstandard. Folgende Werte wurden erhalten.

| TTS gemäß | Permeation mg/2,54 cm$^2$ | |
|---|---|---|
| | nach 8 h | nach 24 h |
| Beispiel 1 | 0,55 | 0,73 |
| Beispiel 2 | 0,45 | 0,741 |
| Beispiel 2a | 0,52 | 1,13 |
| Beispiel 6 (Salbutamol) | 0,04 | 0,11 |

Beispiel 5:

3,98 g festes Harz, Copolymer von Diolefinen und Olefinen
(z.B. Escorez ® 4401)
3,25 g Styrol-Isopren-Styrol-Blockcopolymer
(z.B. Cariflex ® TR 1107)
0,83 g Polyisobutylen
relative mittlere Molekülmasse
ca. 800
(z.B. Oppanol ® B 3)

werden in einer Apparatur entsprechend Beispiel 2a eine Stunde lang bei einer Temperatur von 160°C mit 100 Umdrehungen pro Minute gerührt und anschließend auf ca. 90°C abgekühlt.

0,21 Tulobuterol-Base

werden zugegeben. Die Masse wird anschließend 20 Minuten lang bei 120 °C und 250 Umdrehungen pro Minute weitergerührt und es entsteht eine klare, streichfähige Polymermasse.

Das Material wird entsprechend Beispiel 2 zu Klebepflastern mit einer 15 $\mu$m starken klaren Polyesterfolie als Träger verarbeitet. Die Pflaster zeigen ein optisch einwandfreies, klar durchsichtiges Aussehen und weisen gute Klebeeigenschaften auf der Haut auf.

Beispiel 6:

4,50 g festes Harz, Copolymer von Diolefinen und Olefinen
(z.B. Escorez ® 4401)
15,50 g Styrol-Isopren-Styrol-Blockcopolymer
(z.B. Cariflex ® TR 1107)
-Lösung 19,2 % (g/g) in Benzin -

werden bis zur vollständigen Auflösung des Harzes in einem zylindrischen Glasgefäß (Innendurchmesser 4 cm) gerührt.

0,45 g Salbutamol-Base

werden hinzugefügt und es wird mit einem Magnetrührer weitergerührt. (Der Ansatz führte zu keinem befriedigendem Ergebnis, da der Wirkstoff sich wie erwartet als nahezu unlöslich erwies.) Die noch deutlich agglomerierten Wirkstoffpartikel wurden durch Behandlung in einer Schwingmühle dispergiert - die Dispersion wurde anschließend unter den gleichen Bedingungen, wie in Beispiel 2 beschrieben, ausgestrichen, getrocknet und kaschiert. Es ergab sich ein Flächengewicht der trockenen Klebmasse von 62 g/m$^2$ und damit ein Wirkstoffgehalt von 0,89 mg/2,54 cm$^2$ bzw. 5,63 mg/ 16 cm$^2$.

Die Pflasterklebmasse bleibt auch nach der Trocknung aufgrund dispergierter Wirkstoffpartikel trübe und ist auf der Haut im Vergleich zu Beispiel 2 und 2a nur schwach klebrig.

7

Gesamtwertung der Beispiele

Das erfindungsgemäße System (Beispiele 2, 2a und 5) ist sowohl mit Styrol-1,3-Dien-Styrol-Blockcopolymeren als alleiniger Polymerkomponente (Beispiel 2 und 2a) in klaren, auf der Haut gut selbstklebenden Pflastern herstellbar wie auch unter Zusatz von weiteren Polymeren wie Polyisobutylen (Beispiel 5).

Wie die Beispiele 3 (in-vitro-Freisetzung) und 4 (Permeation durch isolierte Tierhaut) zeigen, weist das erfindungsgemäße Beispiel 2 gegenüber dem Stand der Technik (Beispiel 1 ) eine äquivalente Wirkstoffabgabe auf, das erfindungsgemäße Beispiel 2a eine Erhöhung der Freisetzungsrate um ca. 60 %. Wird dagegen die Rezeptur (ansonsten entsprechend Beispiel 2) statt mit Tulobuterol mit dem schwächer als Tulobuterol wirksamen Wirkstoff Salbutamol hergestellt (Beispiel 6), bleibt die Wirkstoffabgabe auf geringem, therapeutisch wertlosen Niveau.

Die besonderen technischen Vorteile der Erfindung werden in der Eignung für lösungsmittelfreie Herstellungsverfahren deutlich, die in Beispiel 2a und 5 dargestellt ist. Auf diese Weise kann der Wirkstoff Tulobuterol in TTS besonders rationell und im wesentlichen ohne Belastung der Umgebung und Umwelt mit Wirkstoff und Lösemittel verarbeitet werden.

**Patentansprüche**
**Patentansprüche für folgende Vertragsstaaten : AT, BE, CH, DE, DK, FR, GB, IT, LI, LU, NL, SE**

1. Transdermales therapeutisches System mit Tulobuterol oder einem seiner pharmazeutisch verträglichen Salze als Wirkstoff, enthaltend eine im wesentlichen wirkstoffundurchlässige Rückschicht und wenigstens eine den Wirkstoff enthaltende Matrixschicht, gekennzeichnet dadurch, daß die Matrixschicht wenigstens ein Styrol-1,3-Dien-Styrol-Blockcopolymer enthält.

2. Transdermales therapeutisches System nach Anspruch 1, dadurch gekennzeichnet, daß das Styrol-1,3-Dien-Styrol-Blockcopolymer ein Styrol-Butadien-Styrol- oder Styrol-Isopren-Styrol-Blockcopolymer ist.

3. Transdermales therapeutisches System nach Anspruch 1, dadurch gekennzeichnet, daß die Matrixschicht selbstklebend ist.

4. Transdermales therapeutisches System nach Anspruch 1, dadurch gekennzeichnet, daß die wirkstoffhaltige Matrixschicht nicht selbstklebend ist und eine getrennte wirkstofffreie Klebeschicht vorhanden ist.

5. Transdermales therapeutisches System nach Anspruch 1 bis 4, dadurch gekennzeichnet, daß mehrere wirkstoffhaltige Matrixschichten vorhanden sind.

6. Transdermales therapeutisches System nach Anspruch 5, dadurch gekennzeichnet, daß bei mehreren wirkstoffhaltigen Matrixschichten die Wirkstoffkonzentration von Schicht zu Schicht zur Haut hin abnimmt.

7. Transdermales therapeutisches System nach Anspruch 1 bis 3, dadurch gekennzeichnet, daß neben wenigstens einer wirkstoffhaltigen Matrixschicht wenigstens eine wirkstofffreie Matrixschicht vorhanden ist.

8. Transdermales therapeutisches System nach Anspruch 1 bis 4, dadurch gekennzeichnet, daß der Wirkstoff in der Matrix durch vollständige Lösung homogen verteilt ist.

9. Transdermales therapeutisches System nach Anspruch 1 bis 4, dadurch gekennzeichnet, daß der Wirkstoff in der Matrix in feinverteilter Suspension oder in Mikrokapseln vorliegt.

10. Transdermales therapeutisches System nach Anspruch 1,dadurch gekennzeichnet, daß die Matrixschicht Polymere, Harze und ggf. Weichmacher enthält.

11. Verfahren zur Herstellung des transdermalen therapeutischen Systems nach Ansprüchen 1 bis 10, dadurch gekennzeichnet, daß die Bestandteile der Matrixschicht einschließlich Wirkstoff homogen, ggf. durch Auflösen in einem geeigneten Lösemittel, vermischt, die Mischung auf die wirkstoffundurchlässige Rückschicht aufgebracht und das Lösemittel anschließend entfernt wird.

**12.** Verfahren nach Anspruch 11, dadurch gekennzeichnet, daß die homogene Mischung der Bestandteile der Matrixschicht, die ggf. Lösemittel enthält, auf eine kleberabweisende Schutzschicht oder Zwischenschicht aufgebracht wird.

**13.** Verfahren nach Anspruch 11 und 12, dadurch gekennzeichnet, daß die selbstklebende Matrixschicht oder die Matrixschichten von der zunächst als Träger verwendeten kleberabweisend ausgerüsteten Folie, vorzugsweise durch Kaschieren, auf die endgültige Rückschicht übertragen werden.

**14.** Verfahren nach Anspruch 11, dadurch gekennzeichnet, daß die Bestandteile der Matrixschicht(en) und Wirkstoff lösungsmittelfrei durch Anwendung von Wärme homogen miteinander vermischt werden.

**15.** Verfahren nach Ansprüchen 11 bis 14, dadurch gekennzeichnet, daß die homogene Mischung der Matrixschicht(en) durch Aufstreichen oder Extrudieren auf den Träger aufgebracht werden.

**Patentansprüche für folgende Vertragsstaaten : ES, GR**

**1.** Verfahren zur Herstellung eines transdermalen therapeutischen Systems, welches eine im wesentlichen wirkstoffundurchlässige Rückschicht und wenigstens eine den Wirkstoff enthaltenden Matrixschicht aufweist, mit Tulobuterol oder einem seiner pharmazeutisch verträglichen Salze als Wirkstoff, dadurch gekennzeichnet, daß eine homogene Mischung der die wirkstoffhaltige Matrix bildenden folgenden Bestandteile hergestellt wird
   - Grundpolymer aus wenigstens einem Styrol-1,3-Dien-Styrol-Blockcopolymer,
   - Tulobuterol oder eines seiner pharmazeutisch verträglichen Salze,
   - gegebenenfalls Hilfsstoffe wie Polymere, klebrigmachende Harze, Antioxidantien, Weichmacher, Füllstoffe, Lösungsvermittler, Aufschmelzhilfsmittel, Emulsionsförderer,
   und daß die homogene Mischung auf die undurchlässige Rückschicht aufgebracht und gegenenfalls Lösungsmittel entfernt werden.

**2.** Verfahren nach Anspruch 1, dadurch gekennzeichnet, daß der Wirkstoff in einem Lösungsmittel mit den Matrixbestandteilen gemischt wird.

**3.** Verfahren nach Anspruch 1, dadurch gekennzeichnet, daß der Wirkstoff in einer vollständigen Lösung homogen verteilt wird oder in Form einer Suspension oder von Mikrokapseln in die Matrix eingebracht wird.

**4.** Verfahren nach Anspruch 1, dadurch gekennzeichnet, daß eine nicht selbstklebend wirkstoffhaltige Matrixschicht und eine wirkstofffreie Klebeschicht getrennt aufgebracht werden.

**5.** Verfahren nach Anspruch 1, dadurch gekennzeichnet, daß mehrere wirkstoffhaltige Matrixschichten aufgebracht werden, wobei die Wirkstoffkonzentration von Schicht zu Schicht zur Haut hin abnehmen kann.

**6.** Verfahren nach Anspruch 1, dadurch gekennzeichnet, daß neben wenigstens einer wirkstoffhaltigen Matrixschicht wenigstens eine wirkstofffreie Matrixschicht vorhanden ist.

**7.** Verfahren nach Anspruch 1, dadurch gekennzeichnet, daß die homogene, gegebenenfalls lösungsmittelhaltige Mischung der Bestandteile der Matrixschicht auf eine kleberabweisende Schutz- oder Zwischenschicht aufgebracht wird.

**8.** Verfahren nach Anspruch 1, dadurch gekennzeichnet, daß die selbstklebende Matrixschicht oder die Matrixschichten von der zunächst als Träger verwendeten kleberabweisend ausgerüsteten Folie, vorzugsweise durch Kaschieren, auf die endgültige Rückschicht übertragen werden.

**9.** Verfahren nach Anspruch 1, dadurch gekennzeichnet, daß die Bestandteile der Matrixschichten und Wirkstoff lösungsmittelfrei durch Anwendung von Wärme homogen miteinander vermischt werden.

**10.** Verfahren nach Anspruch 1, dadurch gekennzeichnet, daß die homogene Mischung der Matrixschichten durch Aufstreichen oder Extrudieren auf den Träger aufgebracht wird.

**Claims**

**Claims for the following Contracting States : AT, BE, CH, DE, DK, FR, GB, IT, LI, LU, NL, SE**

1. Transdermal therapeutic system with tulobuterol or one of the pharmaceutically acceptable salts thereof as active substance, comprising a backing layer which is substantially impermeable to active substances and at least one matrix layer which contains the active substance, characterized in that the matrix layer comprises at least one styrene-1,3-diene-styrene block copolymer.

2. The transdermal therapeutic system according to claim 1, characterized in that the styrene-1,3-diene-styrene block copolymer is a styrene-butadiene-styrene block copolymer or a styrene-isoprene-styrene block copolymer.

3. The transdermal therapeutic system according to claim 1, characterized in that the matrix layer is self-adhesive.

4. The transdermal therapeutic system according to claim 1, characterized in that the active substance-containing matrix layer is not self-adhesive and a separate, active substance-free adhesive layer is present.

5. The transdermal therapeutic system according to claims 1 to 4, characterized in that several active substance-containing matrix layers are present.

6. The transdermal therapeutic system according to claim 5, characterized in that in the case of several active substance-containing matrix layers, the active substance concentration decreases from layer to layer towards the skin.

7. The transdermal therapeutic system according to claims 1 to 3, characterized in that at least one matrix layer which is free of active substance is present in addition to at least one active substance-containing matrix layer.

8. The transdermal therapeutic system according to claims 1 to 4, characterized in that the active substance is homogeneously dispersed within the matrix by complete dissolution.

9. The transdermal therapeutic system according to claims 1 to 4, characterized in that the active substance is present within the matrix in finely distributed suspension or in microcapsules.

10. The transdermal therapeutic system according to claim 1, characterized in that the matrix layer comprises polymers, resins, and optionally softeners.

11. Process for the production of the transdermal therapeutic system as defined in claims 1 to 10, characterized in that the components of the matrix layer including the active substance are homogeneously mixed, if necessary by means of dissolution in a suitable solvent, the mixture is applied to the backing layer impermeable to active substance, and the solvent is thereafter removed.

12. The process according to claim 11, characterized in that the homogeneous mixture of the components of the matrix layer, which optionally comprises solvents, is applied to an adhesive-repelling protective or intermediate layer.

13. The process according to claims 11 and 12, characterized in that the self-adhesive matrix layer or matrix layers is/are transferred from the adhesive-repellent film or foil which has initially been used as carrier, to the final backing layer, preferably by means of lamination coating.

14. The process according to claim 11, characterized in that the components of the matrix layer(s) and the active substance are homogeneously mixed with each other without the use of solvents, by the application of heat.

15. The process according to claims 11 to 14, characterized in that the homogeneous mixture of the matrix layer(s) is applied to the carrier by spreading or extruding.

**Claims for the following Contracting States : ES, GR**

1. Process for the production of a transdermal therapeutic system having a backing layer which is substantially impermeable to active substance and at least one matrix layer containing the active substance, and comprising tulobuterol or one of its pharmaceutically acceptable salts as the active substance, characterized in that a homogeneous mixture is prepared of the following components forming the active substance-containing matrix:
   - base polymer of at least one styrene-1,3-diene-styrene block copolymer,
   - tulobuterol or one of its pharmaceutically acceptable salts
   - optionally auxiliaries such as polymers, tackifying resins, antioxidants, plasticizers, filling agents, solubilizers, melting auxiliaries, emulsion promoters,
   and that the homogeneous mixture is applied to the impermeable backing layer and, optionally, solvents are removed.

2. The process according to Claim 1, characterized in that the active substance is mixed in a solvent with the matrix components.

3. The process according to Claim 1, characterized in that the active substance is distributed homogeneously in a complete solution, or it is incorporated in the matrix in the form of a suspension or in the form of microcapsules.

4. The process according to Claim 1, characterized in that a non-self-adhesive active substance-containing matrix layer and an active substance-free adhesive layer are applied seperately.

5. The process according to Claim 1, characterized in that several active substance-containing matrix layers are applied, whereby the active substance concentration may decrease from one layer to another towards the skin.

6. The process according to Claim 1, characterized in that, apart from at least one active substance-containing matrix layer, there is present at least one matrix layer which is free of active substance.

7. The process according to Claim 1, characterized in that the homogeneous and, optionally, solvent-containing, mixture of the matrix layer components is applied to an adhesive-repellent protective or intermediate layer.

8. The process according to Claim 1, characterized in that the self-adhesive matrix layer or matrix layers is/are transferred from the adhesive-repellent film or foil which has initially been used as carrier, to the final backing layer, preferably by laminating.

9. The process according to Claim 1, characterized in that the components of the matrix layers and the active substance are homogeneously mixed with one another by application of heat and without using solvents.

10. The process according to Claim 1, characterized in that the homogeneous mixture of the matrix layers is applied to the carrier by spreading or by extrusion.

**Revendications**
**Revendications pour les Etats contractants suivants : AT, BE, CH, DE, DK, FR, GB, IT, LI, LU, NL, SE**

1. Système thérapeutique transdermique avec du tulobutérol ou un de ses sels pharmaceutiquement compatibles, à titre de principe actif, contenant une couche dorsale sensiblement imperméable au principe actif et au moins une couche formant matrice contenant le principe actif, caractérisé en ce que la couche formant matrice contient au moins un copolymère séquencé de styrène-1,3-diène-styrène.

2. Système thérapeutique transdermique selon la revendication 1, caractérisé en ce que le copolymère séquencé de sytrène-1,3-diène-styrène est un copolymère séquencé de styrène-butadiène-styrène ou de styrène-isoprène-styrène.

**3.** Système thérapeutique transdermique selon la revendication 1, caractérisé en ce que la couche formant matrice est autocollante.

**4.** Système thérapeutique transdermique selon la revendication 1, caractérisé en ce que la couche formant matrice contenant le principe actif n'est pas autocollante et une couche de colle dépourvue de principe actif séparée est présente.

**5.** Système thérapeutique transdermique selon la revendication 1, caractérisé en ce que plusieurs couches formant matrice contenant du principe actif sont présentes.

**6.** Système thérapeutique transdermique selon la revendication 5, caractérisé en ce que, dans le cas de la présence de plusieurs couches formant matrice contenant du principe actif, la concentration en principe actif diminue couche en couche en direction de la peau.

**7.** Système thérapeutique transdermique selon l'une quelconque des revendications 1 à 3, caractérisé en ce que, outre au moins une couche formant matrice contenant le principe actif, au moins une couche formant matrice dépourvue de principe actif est présente.

**8.** Système thérapeutique transdermique selon l'une quelconque des revendications 1 à 4, caractérisé en ce que le principe actif est réparti de manière homogène dans la matrice par dissolution totale.

**9.** Système thérapeutique transdermique selon l'une quelconque des revendications 1 à 4, caractérisé en ce que le principe actif est présent dans la matrice sous forme de suspension finement divisée ou dans des microcapsules.

**10.** Système thérapeutique transdermique selon la revendication 1, caractérisé en ce que la couche formant matrice contient des polymères, des résines et éventuellement des plastifiants.

**11.** Procédé de fabrication du système thérapeutique transdermique selon l'une quelconque des revendications 1 à 10, caractérisé en ce que l'on mélange les constituants de la couche formant matrice, y compris le principe actif, de manière homogène, éventuellement par dissolution dans un solvant commun, on applique le mélange sur la couche dorsale imperméable au principe actif et on élimine ensuite le solvant.

**12.** Procédé selon la revendication 11, caractérisé en ce que le mélange homogène des constituants de la couche formant matrice, qui contient éventuellement du solvant, est appliqué sur une couche de protection repoussant la colle, ou une couche intermédiaire.

**13.** Procédé suivant l'une quelconque des revendications 11 et 12, caractérisé en ce que l'on transfère la couche formant matrice autocollante ou les couches formant matrice autocollantes de la feuille rendue répulsive vis-à-vis de la colle, d'abord utilisée comme support, sur la couche dorsale finale, de préférence par lamification ou stratification.

**14.** Procédé selon la revendication 11, caractérisé en ce que l'on mélange mutuellement les constituants de la ou des couches formant matrice et le principe actif sans solvant de manière homogène par l'emploi de chaleur.

**15.** Procédé suivant l'une quelconque des revendications 11 à 14, caractérisé en ce que l'on applique le mélange homogène de la ou des couches formant matrice par étalement ou extrusion sur le support.

**Revendications pour les Etats contractants suivants : ES, GR**

**1.** Procédé de fabrication d'un système thérapeutique transdermique, qui comporte une couche dorsale sensiblement imperméable au principe actif et au moins une couche formant matrice contenant le principe actif, avec du tulobutérol ou un de ses sels pharmaceutiquement compatibles, à titre de principe actif, caractérisé en ce que l'on prépare un mélange homogène des constituants suivants formant la matrice contenant le principe actif
- polymère de base d'au moins un copolymère séquencé de styrène-1,3-diène-styrène,

- tulobutérol ou un de ses sels pharmaceutiquement compatibles,
- éventuellement des adjuvants, comme des polymères, des résines conférant de l'adhésivité, des antioxydants, des plastifiants, des charges, des agents conférant de la solubilité, des adjuvants de fusion, des agents favorisant l'émulsion,

et on applique le mélange homogène sur la couche dorsale imperméable et on en élimine éventuellement le solvant.

2. Procédé selon la revendication 1, caractérisé en ce que l'on mélange le principe actif dans un solvant aux constituants de la matrice.

3. Procédé selon la revendication 1, caractérisé en ce que l'on répartit le principe actif de manière homogène dans une solution complète, ou bien on l'incorpore à la matrice sous la forme d'une suspension ou de microcapsules.

4. Procédé selon la revendication 1, caractérisé en ce que l'on applique séparément une couche formant matrice contenant le principe actif, non autocollante et une couche de colle dépourvue de principe actif.

5. Procédé selon la revendication 1, caractérisé en ce que l'on applique plusieurs couches formant matrice contenant le principe actif, où la concentration en principe actif peut diminuer de couche en couche en direction de la peau.

6. Procédé selon la revendication 1, caractérisé en ce que, outre au moins une couche formant matrice contenant le principe actif, est présente au moins une couche formant matrice dépourvue de principe actif.

7. Procédé selon la revendication 1, caractérisé en ce que le mélange homogène des constituants de la couche formant matrice, contenant éventuellement un solvant, est appliqué sur une couche de protection repoussant la colle ou sur une couche intermédiaire.

8. Procédé selon la revendication 1, caractérisé en ce que la couche formant matrice autocollante ou les couches formant matrice sont transférées de la feuille rendue répulsive vis-à-vis de la colle, d'abord utilisée comme support, à la couche dorsale finale, de préférence par stratification ou lamification.

9. Procédé selon la revendication 1, caractérisé en ce que l'on mélange mutuellement les constituants des couches formant matrice et le principe actif sans solvant, de manière homogène, par utilisation de chaleur.

10. Procédé selon la revendication 1, caractérisé en ce que l'on applique le mélange homogène des couches formant matrice par étalement ou extrusion sur le support.